# EUROPEAN PATENT APPLICATION

(11) **EP 3 318 877 A1**
(43) Date of publication of application: **09.05.2018**
(21) Application number: 17001642.2
(22) Date of filing: 30.07.2015
(51) Int. Cl.: G01N 33/564, G01N 33/92, G01N 33/543

(54) **DIAGNOSTIC IMMUNODETECTION OF AUTOANTIBODIES TO GANGLIOSIDES**

(62) Divisional of application: 15002253.1
(71) Applicant: Euroimmun Medizinische Labordiagnostika AG, 23560 Lübeck (DE)
(72) Inventor: Scheper, Thomas, 23919 Berkenthin (DE); Meyer, Wolfgang, 23689 Pansdorf (DE)

(57) **Abstract**

The present invention relates to a method comprising the steps contacting in a reaction mixture a liquid sample comprising antibodies from a subject with a ganglioside and detecting a complex comprising the ganglioside and an autoantibody to the ganglioside, wherein the first step is carried out in the presence of an isolated ganglioside-binding protein; a reaction mixture comprising a ganglioside, and a liquid sample comprising antibodies from a subject and an isolated ganglioside-binding protein, preferably further comprising an autoantibody to the ganglioside; a complex comprising a ganglioside and an autoantibody to the ganglioside in the presence of an isolated ganglioside-binding protein, wherein the complex is preferably purified; and a use of a ganglioside-binding protein, preferably an isolated, ganglioside-binding protein for increasing the sensitivity of an assay detecting autoantibodies to a ganglioside for the purpose of diagnosing a disease.

## Description

The present invention relates to a method comprising the steps contacting in a reaction mixture a liquid sample comprising antibodies from a subject with a ganglioside and detecting a complex comprising the ganglioside and an autoantibody to the ganglioside, wherein the first step is carried out in the presence of an isolated ganglioside-binding protein; a reaction mixture comprising a ganglioside, and a liquid sample comprising antibodies from a subject and an isolated ganglioside-binding protein, preferably further comprising an autoantibody to the ganglioside; a complex comprising a ganglioside and an autoantibody to the ganglioside in the presence of an isolated ganglioside-binding protein, wherein the complex is preferably purified; and a use of a ganglioside-binding protein, preferably an isolated, ganglioside-binding protein for increasing the sensitivity of an assay detecting autoantibodies to a ganglioside for the purpose of diagnosing a disease.

It is widely accepted that autoantibodies to glycolipids are linked to various neurological diseases such as Guillain-Barre syndrome (GBS), Chronic inflammatory Demyelinating Polyneuropathy (CIPD), Multifocal Motor Neuropathy (MMN) and Miller Fisher syndrome (MFS) and related conditions, and many commercially available assays for the diagnosis of these diseases center around the detection of such autoantibodies in samples. A positive result suggests that the subject is likely to develop or suffer from such a disease (see Willison, H. J., and Yuki, N. (2002): Peripheral neuropathies and anti-glycolipid antibodies, Brain 125, 2591-2625 for a review).

The state of the art teaches a multitude of methods that may be used to detect the diagnostically relevant autoantibodies (see Zane, H. D. (2001), Immunology - Theoretical & Practical Concepts in Laboratory Medicine, W. B. Saunders Company, in particular in Chapter 14). Methods commonly used include immunoblots such as dot blots, Western blots or line blots, ELISA, agglutination assays, surface plasmon resonance and the like.

Assays based on line blots (Raoult, D., and Dasch, G. A. (1989), The line blot: an immunoassay for monoclonal and other antibodies. Its application to the serotyping of gram-negative bacteria. J. Immunol. Methods, 125 (1- 2), 57-65; WO2013041540) are particularly convenient, since they can be carried out and evaluated both in a manual and an automated fashion.

A line blot tailor-made for the detection of autoantibodies against gangliosides is available from EUROIMMUN, Lübeck ("Antibodies against gangliosides (IgG) - Test instruction for the Ganglioside Profile 2 EUROLINE", published on October 29, 2013). This product comprises membrane strips comprising antigens GM1, GM2, GM3, GD1a, GD1b, GT1b and GQ1b. According to the manufacturer's instructions, the test strip should be incubated with diluted samples suspected of containing autoantibodies to ganglioside antigens at room temperature for at least 60 minutes, followed by detection of any bound autoantibodies.

However, a common problem underlying all assays commercially available is the intrinsic instability of reagents and antibodies to be detected. Gangliosides are known as very labile compounds. When kept at room temperature, they lose their antigenic properties owing to conformational changes or chemical instability (Asati, A., Kachurina, O., and Kachurin, A. (2012), Simultaneous Measurements of Auto-Immune and Infectious Disease Specific Antibodies Using a High Throughput Multiplexing Tool. PLOS ONE 7 (8), e42681). Serum anti-ganglioside antibodies have limited thermal stability, which is why the assay is usually performed at 4°C (Willison, H. J. (2007), Ganglioside Autoantibodies. Autoantibodies 611-618), in particular the formation of the antibody-antigen complex.

The thermal instability of the reagents presents users with significant problems. The assay mixture has to be cooled for prolonged periods of time. This is not only inconvenient and requires sophisticated laboratory equipment including a cooling unit and leads to increased energy consumption, whereas a lack of stringent refrigeration, for example due to malfunction or extreme weather or climatic conditions, may compromise the reproducibility and reliability of the test.

The problem underlying the present invention aims to overcome any problems associated with state of the art diagnostic assays based on the detection of autoantibodies to gangliosides.

More specifically, a problem underlying the present invention is to provide a diagnostic assay having a high degree of reliability, in particular in terms of sensitivity, that is yet simple to run in an automated fashion, with as few different steps and changes of conditions as possible. Reliable results should be obtainable with any type of sample including aphaeresis samples.

Another problem underlying the present invention is to provide a diagnostic assay that is fast and energy-efficient and may be carried out where no cooling facilities are available, but is yet reliable, in particular in terms of sensitivity.

Another problem underlying the present invention is to provide a diagnostic assay less dependent on climatic conditions and changes than those described in the state of the art.

Another problem underlying the present invention is to provide a reliable diagnostic assay that does not require any change of temperature, in particular any cooling following preparation of the assay reagents and samples.

The problem underlying the present invention is solved by the subject-matter of the attached independent and dependent claims.

In a first aspect, the problem underlying the present invention is solved by a method comprising the steps
a) contacting in a reaction mixture a liquid sample comprising antibodies from a subject with a ganglioside and
b) detecting a complex comprising the ganglioside and an autoantibody to the ganglioside,
wherein step a) is carried out in the presence of an isolated ganglioside-binding protein.

In a second aspect, the problem underlying the present invention is solved by a reaction mixture comprising a ganglioside and a liquid sample comprising antibodies from a subject as well as an isolated ganglioside-binding protein, preferably further comprising an autoantibody to the ganglioside.

In a third aspect, the problem underlying the present invention is solved by a complex comprising a ganglioside and an autoantibody to the ganglioside in the presence of an isolated ganglioside-binding protein, wherein the complex is preferably isolated.

In a preferred embodiment of the third aspect of the invention, the medical device comprises this complex in the presence of an isolated ganglioside-binding protein.

In a 4th aspect, the problem underlying the present invention is solved by a use of a ganglioside-binding protein, preferably an isolated, ganglioside-binding protein, for increasing the diagnostic reliability, preferably sensitivity of an assay detecting autoantibodies to a ganglioside for the purpose of diagnosing a disease.

In a preferred embodiment of any aspect of the present invention, the ganglioside is selected from the group comprising GM1, GM2, GM3, GD1a, GD1b, GT1a, GT1b and GQ1b.

In a preferred embodiment of any aspect of the present invention, the ganglioside-binding protein is an albumin, preferably a mammalian albumin or a variant thereof, more preferably bovine serum albumin or a variant thereof.

In a preferred embodiment of any aspect of the present invention, the ganglioside-binding protein is present at a concentration of 0.001 to 5%, more preferably 0.005 to 1 %, more preferably 0.01 to 1 %.

In a preferred embodiment of any aspect of the present invention, the method, preferably step a) is performed, or the reaction mixture, complex or medical device is at or kept at a temperature between 15 to 37 °C, preferably 18 to 34 °C, more preferably 18 to 30 °C.

In a preferred embodiment of the preceding aspect of the present invention, the reaction mixture is kept, while the method, preferably step a), is performed, at a temperature of at least 10, more preferably 12, 15 or 18 °C at all times.

In a preferred embodiment of any aspect of the present invention, the ganglioside is immobilized, preferably on a medical device.

In a preferred embodiment of any aspect of the present invention, the liquid sample is contacted with or the medical device comprises, preferably in an immobilized form, two or more, preferably three or more, preferably all gangliosides from the group comprising GM1, GM2, GM3, GD1a, GD1b, GT1a, GT1b and GQ1b.

In a preferred embodiment of any aspect of the present invention, the method, reaction mixture, complex or medical device is for the diagnosis of a neurological disease, preferably a neuropathy, more preferably selected from the group comprising GBS, CIPD, MMN and MFS.

In a preferred embodiment, the medical device is selected from the group comprising a test strip, a bead, a microtiter plate, a membrane, and a blot.

In a preferred embodiment of any aspect of the present invention, the complex is detected using a method from the group comprising determining radioactivity, enzymatic activity, in particular colourimetric detection of enzymatic activity, fluorescence, spectroscopy, in particular UV/vis, EPR or NMR spectroscopy, agglutination or chemiluminescence.

The present invention is based on the inventors' surprising finding that the reliability of the assay is improved if ganglioside-binding proteins are present as the antigen is contacted with the sample comprising antibodies. The inventors theorize that contact with a ganglioside-binding protein induces that gangliosides, in particular ganglioside coated on a test strip, adopt a conformation associated with an increased affinity to diagnostically relevant autoantibodies.

It is essential that the sample used to practice the present invention comprises antibodies, also referred to as immunoglobulins. Typically the sample is a bodily fluid comprising a representative set of the entirety of the subject's immunoglobulins. However, the sample, once provided, may be subjected to further processing which may include fractionation, centrifugation, enriching or isolating the entirety of immunoglobulins or any immunoglobulin class of the subject, which may affect the relative distribution of immunoglobulins of the various classes. The sample may be selected from the group comprising whole-blood, serum, cerebrospinal fluid and saliva and is preferably serum.

The method according to the present invention comprising the steps a) contacting in a reaction mixture a liquid sample comprising antibodies from a subject with a ganglioside and b) detecting a complex comprising the ganglioside and an autoantibody to the ganglioside, wherein step a) is carried out in the presence of an isolated ganglioside-binding protein. Step a) requires setting up the reaction mixture including the sample comprising antibodies and the ganglioside and incubating it at a temperature, under conditions compatible with binding of the autoantibody to the ganglioside long enough to allow for formation of the complex, for example, for at least 10, 15, 30, 45, 60, 90, 120, 240 minutes or overnight, preferably for at least 30 minutes, more preferably at least 60 minutes. Subsequently, any complex formed may be separated from the remaining of the sample, for example by removing the liquid reaction mixture from an immobilized ganglioside. This may be followed, for increased reliability, by removal of remaining sample by one or more washing steps using a washing buffer that may comprise the ganglioside-binding protein. Finally, any complex may be detected using various methods described in the state of the art.

According to the present invention, the method, in particular step a) is carried out in the presence of an isolated ganglioside-binding protein. In a preferred embodiment, the term "isolated" ganglioside-binding protein refers to a ganglioside-binding protein that is not an endogenous ganglioside-binding protein from the subject who donated the sample and has been added to the sample either while or prior to setting up the reaction mixture. In a more preferred embodiment, the "isolated" ganglioside-binding protein is a protein purified from a tissue including blood, preferably a mammalian tissue, or using recombinant methods such that it was at least 30, 50, 75, 80, 90, 95 or 99% pure prior to addition to the sample, preferably as judged by visual inspection of an SDS PAGE gel following Coomassie staining. In another preferred embodiment, the isolated ganglioside-binding protein is from another organism than the sample analyzed. In a preferred embodiment the ganglioside-binding protein constitutes at least 30, 50, 75, 80, 90, 95 or 99% of the total protein isolated present in the sample during step a).

The reaction mixture is an aqueous solution that provides conditions compatible with formation of an antigen-antibody complex, more preferably a complex comprising an autoantibody to a ganglioside and a ganglioside. Suitable conditions have been described in the state of the art and comprise, for example, phosphate buffered saline buffer.

The complex comprising a ganglioside and an autoantibody to the ganglioside may be obtained using methods described in the state of the art for the purification of such complexes. The autoantibody or complex may be purified from samples taken from patients suffering from a neurological disease comprising GBS, CIPD, MMN and MFS. In a preferred embodiment, the term "purified", as used herein, means that the complex is enriched such that, compared to its endogenous environment, a smaller amount of proportion of other polypeptides, more preferred antibodies, are present. In a most preferred embodiment, this means that the complex is at least 50, preferably, 60, 70, 80, 90 or 99 % pure as judged by SDS PAGE followed by Coomassie staining and visual inspection.

Within the scope of the present invention does a medical or diagnostic device comprise, preferably coated with one or more than one ganglioside. Preferably such a medical or diagnostic device comprises the ganglioside in a form that allows contacting it with an aqueous solution, more preferably the liquid human sample, in a straightforward manner. In particular, the ganglioside may be immobilized on the surface of a device. Preferably the device is selected from the group comprising glass plates or slides, biochips, microtiter plates, beads, for example magnetic beads, aphaeresis devices, chromatography columns, membranes or the like. Exemplary medical devices include line blots, microplates, and glass slides for microscopy, beads and biochips. In addition to the inventive polypeptide, the medical or diagnostic device may comprise one or more than one additional antigen, one or more than one control such as a positive or negative control or one or more than one known antigen related to other diseases associated with one or more identical or similar symptoms. A control may be a positive control confirming that serum or a diagnostic reagent has been added to the assay.

In a preferred embodiment the invention contemplates a use of a ganglioside-binding protein, preferably an isolated, ganglioside-binding protein for increasing the diagnostic reliability, preferably sensitivity of an assay detecting autoantibodies to a ganglioside for the purpose of diagnosing a disease. The assay may comprise the steps to be carried out in line with the inventive method. Moreover, the assay, more preferably step a) may be performed at a temperature between 15 to 37 °C, preferably 18 to 34 °C, more preferably 18 to 30 °C and/or kept at a temperature of at least 10, more preferably 12, 15 or 18 °C at all times, wherein in a preferred embodiment the term "at all times" refers to the period starting with with the preparation of the assay reagents and materials, more preferably the period starting with the initial contact of sample and ganglioside, more preferably the period between initial contact of sample and ganglioside and addition of a reagent for the detection of the complex comprising ganglioside and autoantibody, most preferably the period between initial contact of sample and ganglioside and removal of the sample.

Preferably the ganglioside is selected from the group comprising GM1, GM2, GM3, GD1a, GD1b, GT1a, GT1b and GQ1b, preferably, GM1. In a more preferred embodiment, a combination of two or more, three or more, four or more, five or more or the entirety of the gangliosides selected from the group comprising GM1, GM2, GM3, GD1a, GD1b, GT1a, GT1b and GQ1b is used.

The ganglioside-binding protein is a protein that is capable of binding to a ganglioside selected from the group comprising GM1, GM2, GM3, GD1a, GD1b, GT1a, GT1b and GQ1b, preferably, GM1, which binding may be detected by gel filtration and UV detection at 280 nm, the detection method being replaceable, depending on the absorption properties of the protein, by subjecting collected fractions to SDS PAGE and silver staining, preferably using the methodology and under the conditions described by Tomas, M., Road, L. G., Ausiello, C., D'Agnolo, G., Venerando, B., Ghidoni, R., Sonnino, S., and Tettamanti, G. (1980) Interaction of GM1 Ganglioside with Bovine Serum Albumin Formation and Isolation of Multiple Complexes, Eur. J. Biochem. 111, 315-324, page 317.

In a preferred embodiment, the isolated ganglioside-binding protein, preferably albumin or a variant thereof, more preferably mammalian albumin or variant thereof, preferably bovine serum albumin (BSA, most preferably BSA represented by data base code CAA76847.1, which, as all data base codes referred to throughout this application, refers to the NCBI data bases as online at the priority date of this application) or variant thereof, is present at a concentration of 0.001 to 5 %, more preferably 0.005 to 1 %, more preferably 0.01 to 1 % (weight/volume), wherein optionally one or more than one other isolated ganglioside-binding protein may replace part of the isolated ganglioside-binding protein. Preferably the concentration is measured by SDS-PAGE of the sample followed by Coomassie staining and visual comparison to bands of known quantities of the protein or proteins or a variant thereof loaded and run on the same gel.

However, the teachings of the present invention may not only be carried out using polypeptides, in particular BSA having the exact sequences referred to in this application explicitly, for example by function, name, sequence or accession number, or implicitly, but also using variants of such polypeptides or nucleic acids.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 25, 50, 75, 100, 150 or 200 successive amino acids of the original sequence or a variant thereof. The total length of the variant may be at 25, 30, 40, 50, 60, 70, 80, 90, 100 or more amino acids.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind to a ganglioside, or the fold or structure of the polypeptide are deleted or substituted and/or one or more such essential amino acids are replaced in a conservative manner and/or amino acids are added such that the biological activity of the polypeptide is preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default settings.

In a preferred embodiment, variants may, in addition, comprise chemical modifications, for example isotopic labels or covalent modifications such as glycosylation, phosphorylation, acetylation, decarboxylation, citrullination, hydroxylation and the like. The person skilled in the art is familiar with methods to modify polypeptides. Any modification is designed such that it does not abolish the biological activity of the variant.

Moreover, variants may also be generated by fusion with other known polypeptides or variants thereof and comprise active portions or domains, preferably having a sequence identity of at least 70, 75, 80, 85, 90, 92, 94, 95, 96, 97, 98 or 99 % when aligned with the active portion of the reference sequence, wherein the term "active portion", as used herein, refers to an amino acid sequence, which is less than the full length amino acid sequence.

The variant of the polypeptide has biological activity. In a preferred embodiment such biological activity is the ability to bind to a ganglioside, preferably such that it is, more preferably when immobilized, better suited to the detection of an autoantibody in a sample.

It is preferred that the inventive method, preferably step a) is performed or the inventive devices and reagents are at room temperature, preferably kept at room temperature, more preferably at a temperature between 10 to 40 °C, preferably 15 to 37 °C, preferably 18 to 34 °C, more preferably 18 to 30 °C, most preferably 20 to 25 °C. In a preferred embodiment, steps a) and b) are carried out without any refrigeration relative to the room temperature, more preferably in the absence of a cooling unit. In a preferred embodiment, the term "room temperature", as used herein, is the routine temperature of the place where the assay is performed and is more preferably 20 to 25 °C, most preferably 20 °C.

In another preferred embodiment, the method, preferably step a), is performed or the inventive devices and reagents are at room temperature, preferably kept at a temperature of at least 10, more preferably 12, 15 or 18 °C at all times. In a more preferred embodiment, in this context step a) starts with the initial contact between the sample and the ganglioside and is concluded with the addition of an analytical reagent binding to the antibody binding to the ganglioside, more preferably with the partial or full removal of the sample.

The objective of the inventive method, reagents and uses may be to provide diagnostically relevant information, more specifically the determination of the presence of antibodies, preferably autoantibodies to a ganglioside in a sample from a patient. The patient may have other symptoms, in particularly clinical symptoms suggesting that they may suffer from a neurological disease. The state of the art teaches how such information may be used for the diagnosis of a neurological disease, for example Willison, H. J., and Yuki, N. (2002): Peripheral neuropathies and anti-glycolipid antibodies, Brain 125, 2591-2625.

In a preferred embodiment, the term "diagnosis", as used herein, refers to any kind of procedure aiming at obtaining information instrumental in the assessment whether a patient suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient with regard to a certain treatment, for example the administration of immunosuppressive drugs. In other words, the term "diagnosis" comprises not only diagnosing, but also prognosticating and/or monitoring the course of a disease or disorder.

The neurological disease is preferably a neuropathy, more preferably selected from the group comprising GBS, CIPD, MMN and MFS, most preferably GBS.

In a preferred embodiment, the ganglioside is an immobilized ganglioside. This way, the immobilized ganglioside, together with the insoluble carrier to which it is attached, may be separated from a reaction mixture in a straightforward manner, for example by filtration, centrifugation or decanting. An immobilized ganglioside may be immobilized in a reversible or irreversible manner. For example, the immobilization is reversible if the molecule interacts with the carrier via ionic interactions which may be masked by addition of a high concentration of salt or if the molecule is bound via a cleavable covalent bond. By contrast, the immobilization is irreversible if the molecule is tethered to the carrier via a covalent bond that cannot be cleaved in aqueous solution. The ganglioside may be indirectly immobilized, for example by immobilizing an antibody or other entity having affinity to the ganglioside, followed by addition of the ganglioside formation of a complex to the effect that the ganglioside -antibody complex is immobilized. The person skilled in the art is familiar with methods for immobilizing molecules such as gangliosides. An exemplary way is described in Chabraoui, F., Derrington, E. A., Mallie-Didier, F., Canfavreux, C., Qincy, C., and Caudie, C. (1993) Dot-blot immunodetection of antibodies against GM1 and other gangliosides on PVDF-P membranes, J. Immun. Meth. 165, 225-230.

In a preferred embodiment, a microplate, membrane, dot blot, or line blot is used to carry out the diagnostic method according to the invention. In a more preferred embodiment, a blot-based method is used, most preferably a line blot. The person skilled in the art is familiar with the experimental setup, which is described in the state of the art. In a preferred embodiment, the term "line blot", as used herein, refers to a test strip that has been coated with one or more band of antigen purified, preferably to at least 80, 90, 95 or 99 % purity, prior to the coating procedure. If two or more antigens are used, they are preferably spatially separated. Preferably, the width of the bands is at least 30, more preferably 40, 50, 60, 70 or 80 % the width of the test strip. The test strip may comprise one or more control bands for confirming that it has been contacted with sample sufficiently long and under sufficient conditions, in particular human serum, antibody conjugate, or both.

Any data demonstrating the presence or absence of the complex comprising the antibody and the inventive polypeptide may be correlated with reference data. For example, detection of said complex indicates that the patient who provided the sample analyzed has suffered, is suffering or is likely to suffer in the future from a disease. If a patient has been previously diagnosed and the method for obtaining diagnostically relevant information is run again, the amount of complex detected in both runs may be correlated to find out about the progression of the disease and/or the success of a treatment. For example, if the amount of complex is found to increase, this suggests that the disorder is progressing, likely to manifest in the future and/or that any treatment attempted is unsuccessful.

The inventive teachings provide a kit, preferably for diagnosing a disease. Such a kit may comprise instructions detailing how to use the kit and a means for contacting the inventive polypeptide with a bodily fluid sample from a subject, preferably a human subject, for example a line blot, wherein the inventive polypeptide is immobilized on the line blot. Furthermore, the kit may comprise a positive control, for example a batch of autoantibody or recombinant antibody known to bind to the inventive polypeptide, and a negative control, for example a protein having no detectable affinity to the inventive polypeptide such as bovine serum albumin. Finally, such a kit may comprise a standard solution comprising a ganglioside-binding antibody for preparing a calibration curve. In a preferred embodiment, the kit comprises a device, preferably a blot-based device such as a line blot coated with a ganglioside and an incubation buffer, optionally for diluting the sample, which comprises a ganglioside-binding protein.

The present invention is further illustrated by the following figures and non-limiting examples from which further features, embodiments, aspects and advantages of the present invention may be taken.

### Example:

Samples from a cohort of 32 patients suffering from a neurological disease characterized by the presence of autoantibodies to gangliosides were subjected to analysis using the EUROIMMUN Ganglioside Profile 2 EUROLINE system, which is commercially available.

The EUROIMMUN Ganglioside Profile 2 EUROLINE provides a qualitative *in vitro* assay for the determination of human ganglioside autoantibodies of class IgG or IgM in the serological diagnosis of inflammatory peripheral neuropathies. By using a combination of different purified antigens (GM1, GM2, GM3, GD1a, GD1b, GT1b and GQ1b) on one strip, multiple ganglioside autoantibodies can be investigated simultaneously in one sample. The EUROIMMUN line blot is designed for processing at room temperature (RT).

Test performance in line with the manufacturer's instructions at RT (20 °C) was compared to performance at 4 °C under otherwise identical conditions. Band intensities were evaluated using the EUROLINEScan software.

Among the 32 samples, two samples obtained by plasmapharesis were identified as containing autoantibodies only when the assay was run at 4 °C. No autoantibody to a ganglioside was detected in one of these samples following incubation at RT.

Three samples obtained by aphaeresis, among them one of the discrepant samples, were then characterized using the same assay in two experimental settings in parallel. Again, the manufacturer's instructions were followed except that a buffer comprising 0.05 % of isolated Bovine Serum Albumin was used as the incubation buffer in the second experimental setting.

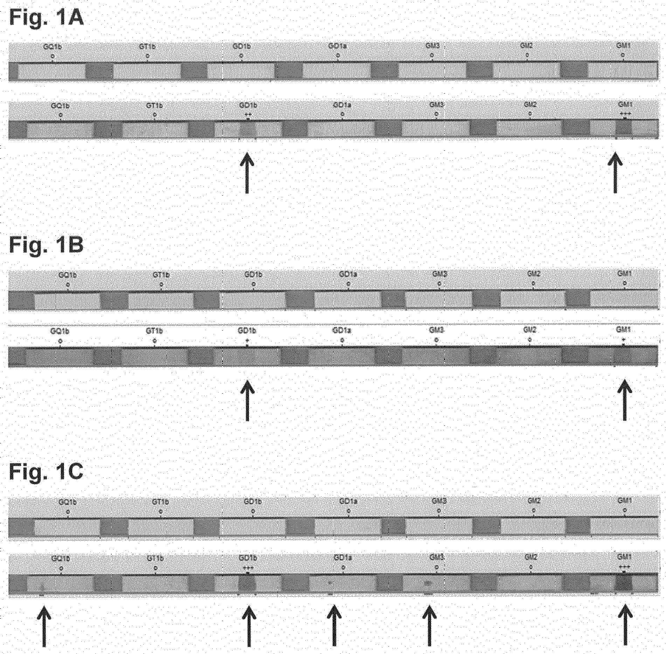

It turned out that autoantibodies to gangliosides could be detected when the buffer comprising BSA was used rather than the conventional buffer. **Figs. 1A, 1B** and **1C** show the primary data obtained using the three samples. In each figure, the respective upper panel represents the line blot following incubation in conventional incubation buffer as provided by the manufacturer, which did not contain an isolated ganglioside-binding protein. The lower panel shows the line blot following incubation in buffer comprising 0.05 % of isolated BSA. Black arrows mark visible bands on each line blot.

## Claims

1. A method comprising the steps
a) contacting in a reaction mixture a liquid sample comprising antibodies from a subject with a ganglioside and
b) detecting a complex comprising the ganglioside and an autoantibody to the ganglioside,
wherein step a) is carried out in the presence of an isolated ganglioside-binding protein.

2. A reaction mixture comprising a ganglioside, and a liquid sample comprising antibodies from a subject as well as an isolated ganglioside-binding protein, preferably further comprising an autoantibody to the ganglioside.

3. A complex comprising a ganglioside and an autoantibody to the ganglioside in the presence of an isolated ganglioside-binding protein, wherein the complex is preferably purified.

4. A medical device comprising the complex according to claim 3 in the presence of an isolated ganglioside-binding protein.

5. A use of a ganglioside-binding protein, preferably an isolated, ganglioside-binding protein for increasing the diagnostic reliability of an assay detecting autoantibodies to a ganglioside for the purpose of diagnosing a disease.

6. The method, reaction mixture, complex, medical device or use according to any of claims 1 to 5, wherein the ganglioside is selected from the group comprising GM1, GM2, GM3, GD1a, GD1b, GT1a, GT1b and GQ1b.

7. The method, reaction mixture, complex, medical device or use according to any of claims 1 to 6, wherein the ganglioside-binding protein is an albumin, preferably a mammalian albumin or a variant thereof, more preferably bovine serum albumin or a variant thereof.

8. The method reaction mixture, complex, medical device or use according to any of claims 1 to 7, wherein the ganglioside-binding protein is present at a concentration of 0.001 to 5 %, more preferably 0.005 to 1 %, more preferably 0.01 to 1 %.

9. The method, reaction mixture, complex or medical device according to any of claims 1 or 4 to 6, wherein the method, preferably step a) is performed or kept at a temperature between 15 to 37 °C, preferably 18 to 34 °C, more preferably 18 to 30 °C.

10. The method according to claim 9, wherein the reaction mixture is kept, while the method, preferably step a), is performed or the reaction mixture, complex or medical device is kept at a temperature of at least 10, more preferably 12, 15 or 18 °C at all times.

11. The method, reaction mixture, complex or medical device according to any of claims 1 to 10, wherein the ganglioside is immobilized, preferably on a medical device.

12. The method, reaction mixture, complex, medical device or use according to any of claims 1 to 11, wherein the liquid sample is contacted with or the medical device comprises, preferably in an immobilized form, two or more, preferably three or more, preferably all gangliosides from the group comprising GM1, GM2, GM3, GD1a, GD1b, GT1a, GT1b and GQ1b.

13. The method, reaction mixture, complex or medical device according to any of claims 1 to 12, wherein the method, reaction mixture, complex or medical device is for the diagnosis of a neurological disease, preferably a neuropathy, more preferably selected from the group comprising GBS, CIPD, MMN and MFS.

14. The method, reaction mixture, complex or medical device according to any of claims 4 to 13, wherein the medical device is selected from the group comprising a test strip, a bead, a microtiter plate, a membrane, and a blot.

15. The method, reaction mixture, complex or medical device according to any of claims 1 to 14, wherein the complex is detected using a method from the group comprising determining radioactivity, enzymatic activity, in particular colourimetric detection of enzymatic activity, fluorescence, spectroscopy, in particular UV/vis, EPR or NMR spectroscopy, agglutination or chemiluminescence.
